# EUROPEAN PATENT APPLICATION

(11) **EP 3 278 772 A1**
(43) Date of publication of application: **07.02.2018**
(21) Application number: 17001317.1
(22) Date of filing: 01.08.2017
(51) Int. Cl.: A61F 5/30

(54) **THERAPEUTIC DEVICE FOR REDUCING THE PAIN AND INFLAMMATION CAUSED BY THE BUNION**

(30) Priority: 02.08.2016 IT 201600081375
(71) Applicant: Pinna, Marco, 16134 Genova (GE) (IT)
(72) Inventor: Pinna, Marco, 16134 Genova (GE) (IT)
(74) Representative: Leucci, Lidia

(57) **Abstract**

Therapeutic device (1 a, 1 b) to reduce pain and inflammation of bunion inside the shoe, comprising a plate-like body (10), in a flexible and elastically deformable material, formed as a substantially rectangular trapezium, where the minor base is replaced by a concave arc (401) having a given radius of curvature, said body (10) being intended to be coupled to the patient's foot by means of a layer (11) of adhesive material, of much lower thickness than the said body (10), connected to the base wall (111), while the top wall (101) is designed for contact with the shoe, being located in use the ends of said device provided with the arch (401) in proximity the joint of the first metatarsal with the big toe, while the square angle of the said body (10) is facing towards the neck of the foot.

## Description

The present invention relates to therapeutic devices, and in particular to devices for orthopedic use. More particularly, the present invention relates to a device for reducing the pain and inflammation caused by the bunion inside of the shoe.

The bunion is a deformation of the foot constituted by the displacement of the head of the first metatarsal from the other; bunion affects predominantly women in adulthood. The causes of this affliction can be multiple; in general may depend on a congenital tendency to tendon laxity, often associated with the use of tight footwear, which push the load on the forefoot.

The solution, in the most serious cases, is usually surgical intervention, although early diagnosis may be associated with proper prophylaxis, which involves firstly the use of more comfortable footwear, and may provide for the use of corrective devices that promote the correct posture recovery to allow the progressive decrease in swelling caused by tissue inflammation, and the pain associated therewith.

US2010 / 0268632 describes a device for the corrective treatment of the bunion; however, this device, which has interesting features from the point of view of action on the cause of the affection, does not prevent the occurrence of swelling at all, nor does it help to reduce the pain caused by the affection. In addition, there is widespread opinion in the orthopedic field, and particularly in the podological field, that devices that want to correct deformity are ineffective because it is impossible to counteract constant and tonic forces, such as those of tendons that maintain the articulation in that position. Sometimes occurs that the devices that want to correct the deformity, they are actually more problematic because they try to modify a structural remedy that the body has slowly developed over the years.

Numerous devices are also known to protect the foot with bunion and, in most cases, have the disadvantages listed below:
(A) the devices on the market are generally ambidextrous and therefore standardized, although two big toes of the same individual are never the same, the more among different subjects;
(B) they have a standard thickness and do not take into account the differences between the footwear, nor how much the big toe which is going to fit it could be deviated, nor what support and posture may have the subject on that day;
(C) generally cover the contact point between the exostosis and the shoe thereby increasing the compression between the bone and the upper;
(D) the devices that cover the exostosis are not blocked on the foot and, if a gap and/or a micro-motion between the device and the foot take place (moccasin, weakly laced shoe, or an oversized shoe), they rub the skin creating reddening and in the most severe and repeated cases bladders, infections and / or ulcers.

The aim of the present invention is to provide a device which is able to relieve pain caused by the bunion and to reduce the inflammation and swelling due to exostosis, which is of a simple structure, so that it can be realized in a disposable manner and in different formats, adapted to meet the different needs of the user, but at the same time presenting a specific shape for each of the two feet. In addition, the device must be easy connectable to the foot, and must adapt naturally to foot and shoe position, without creating any further undesirable pressure, especially on exostosis.

The object of the present invention is a therapeutic device for reducing the pain and inflammation of the bunion inside the shoe, comprising a plate-like body, in a flexible and elastically deformable material, formed as a substantially rectangular trapezium, where the minor base is replaced by a concave arc having a given radius of curvature, said body being intended to be coupled to the patient's foot by means of a layer of adhesive material of a much smaller thickness than that of said body connected to the base wall, while the top wall is intended for contact with the shoe, the end of said device having the arch being placed in use near the articulation of the first metatarsal with the toe while the right angle of said body is facing the neck of the foot.

In an embodiment of the present invention, the material used to make said body is felt based upon animal hair, of half-compressed type and having a thickness comprised between 4,0 mm and 12,0 mm. More preferably, the thickness of the said body is comprised between 5,0 mm and 7,0 mm. In a different embodiment, the thickness is increasing from the end provided with the arch toward the opposite end.

In a further embodiment the side walls of the said body are preferably tapered, and more preferably inclined in respect to the axis perpendicular to the base wall, of an angle α comprised between 30° and 45°.

The radius of curvature R of the arc segment is about 30% of the oblique side of said trapezoidal rectangle.

A further object of the present invention is the series of therapeutic devices made for the right foot and the left foot of the patient, in which the oblique side of trapezoidal rectangle corresponds substantially to the distance between the middle of plantar arch of the patient's foot and the articulation of the first metatarsal with the big toe, the main base corresponds to approximately 80% of this distance, the side that concurs to form the square angle corresponds to about 95% of that distance, this distance being parameterized on the basis of the usual sizes of footwear on the market, so as to provide a plurality of different dimensions of the device according to the invention.

Further advantages and features will become evident from the following description of an embodiment of the present invention provided, as a non limiting example, with reference to the appended tables of drawings, in which:
Fig. 1 is a perspective view of an embodiment of a device according to the present invention;
Fig. 2 is a top plan view showing two devices according to the present invention, intended to the use on each respective foot of a patient; and
Fig. 3 is schematic view of a device according to the present invention positioned on the foot of the patient.

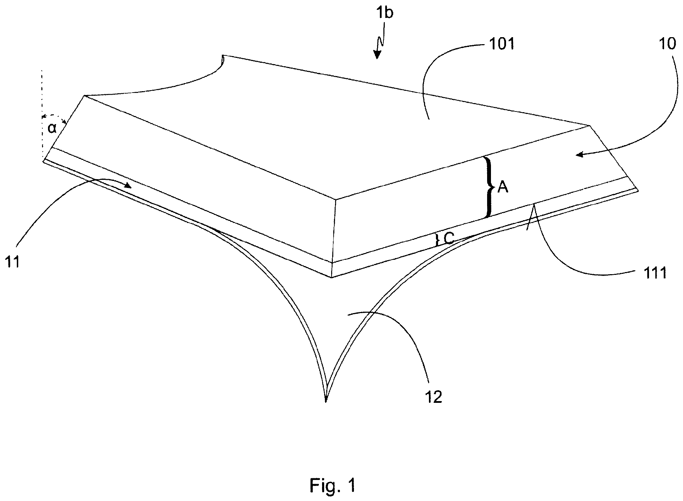

Figure 1 shows an embodiment of the device according to the present invention, indicated by the numeral 1 b; said device comprises a body of elastically compressible material, in the particular case a felt made of animal hair, slab and formed essentially into a truncated pyramid. The body has a base wall 111 coupled to a layer of adhesive material 11; the thickness C of the adhesive layer 11 is much smaller than the thickness A of the body 10, and the adhesive material is protected, on its face opposite to that connected to the body 10, by a layer of protective material of a thickness which is irrelevant to the thickness of the body and of the adhesive material itself. The side walls of the body are inclined, with respect to the axis perpendicular to the base wall 111, of an angle α.

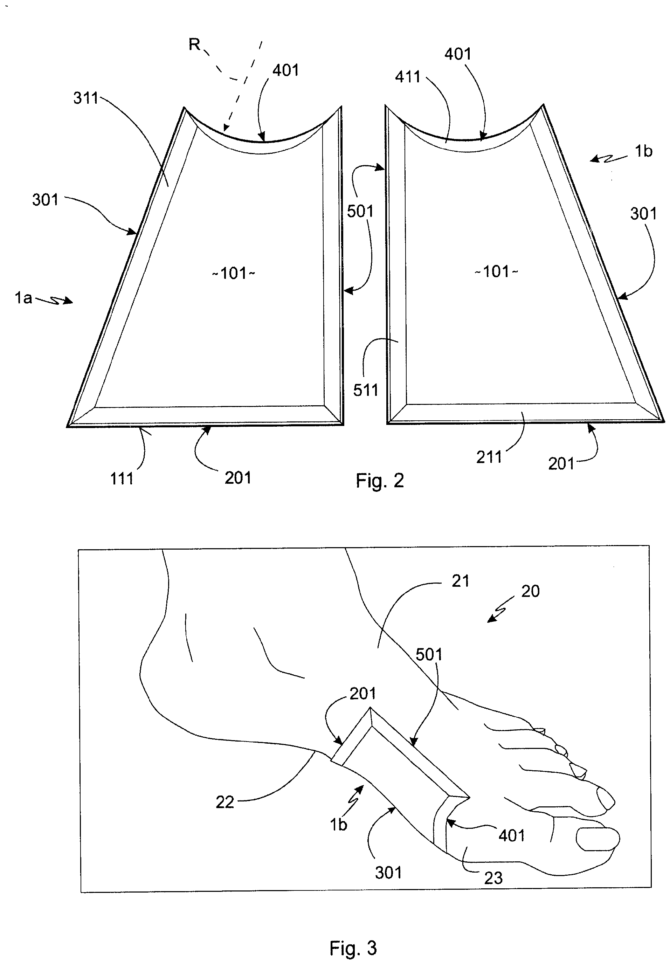

Figure 2 shows two devices, designated 1 a and 1 b, which exhibit the same structural and morphological characteristics except that they are mirrored in one another; device 1 b is intended to be applied to the left foot of the patient, as will be apparent below, while device 1 a is intended for the patient's right foot. The devices are rectangular trapezoidal, and the minor base of said trapezium is replaced by a circular arc segment 401 with a radius of curvature R. The main base 201 is about 80% in length with respect to the oblique side 301, while the side 501 which concurs to the formation of the square angle with the base 201 is approximately about 95% of the same oblique side. The radius of curvature R is about 30% relative to said oblique side 301. As already shown in Figure 1, the side walls 211, 311, 511 and 411 of the body 10 are tapered from the base wall 111 to the top wall 101.

Figure 3 illustrates the device 1 b of Figures 1 and 2, applied to a left foot 20. As can be seen, the end bearing the arch 401 is arranged at the first metatarsus so as to leave the articulation 23 uncovered, and the square angle between sides 201 and 501 is facing the neck of foot 21. The oblique side 301 actually covers substantially half the length of the foot arch 22.

The operation of the device according to the present invention will be apparent from the following. As pointed out in the premise, one of the main problems in the treatment of the bunion lies in the fact that most of the devices used to bridge the contact between the exostosis and the shoe cover the exostosis itself, thus increasing its compression, and consequently the pain and inflammation. The shape studied for the device according to the present invention allows instead to create a thickness immediately downstream of the exostosis itself towards the arch of the foot, thus keeping away the footwear from the articulation, thereby reducing the swelling of the same and the pain resulting from it.

This device, which was born through a research based on podiatric practice, is thus realized taking into account the structural parameters of the foot of the patient; series of devices, conceived for both the right foot and the left foot, will be realized, in which the reference dimension is the oblique side 501, which substantially identifies half of the length of the arch of the foot from the articulation 23 of the first metatarsal with the big toe. The other elements of the trapezoidal body 10 of the device will be defined accordingly, maintaining the proportions described above. The series of devices made will be able to take into account the common footwear measurements, as can be achieved with reference to the average statistic size of the feet to be treated.

Preferably used material is felt; the choice of this material comes from its consolidated use in the podiatric field, and is linked to both its mechanical characteristics and its complete compatibility with the epidermis. The felt used to make the body 10 will be of thickness A between 6.0 mm and 12.0 mm, and preferably will be 10.0 mm. In an embodiment not shown, the body 10 has a uniformly decreasing thickness from the end bearing the right angle to the end carrying the arc segment. The layer of adhesive material will be made of adhesive material suitable for use on the human epidermis, and preferably it can be made of silicone rubber or similar materials suitable for use.

The tapering on the side walls is preferable but is not strictly necessary to achieve the objects of the invention according to the present invention. The inclination of the side walls of the body 10 is designed to reduce the friction between the wall 101 of the body 10, and in particular the edges between the latter and the side walls, and the shoe worn by the patient. The angle α will preferably be between 30 ° and 45 ° with respect to the axis perpendicular to the wall 101 and to the wall 111.

## Claims

1. Therapeutic device (1a, 1b) to reduce pain and inflammation of bunion inside the shoe, comprising a plate-like body (10), in a flexible and elastically deformable material, formed as a substantially rectangular trapezium, where the minor base is replaced by a concave arc (401) having a given radius of curvature, said body (10) being intended to be coupled to the patient's foot by means of a layer (11) of adhesive material, of much lower thickness than the said body (10), connected to the base wall (111), while the top wall (101) is designed for contact with the shoe, being located in use the ends of said device provided with the arch (401) in proximity the joint of the first metatarsal with the big toe, while the square angle of the said body (10) is facing towards the neck of the foot.

2. Device according to claim 1, wherein the material used to make said body (10) is felt based upon animal hair.

3. Device according to claim 1 or 2, wherein said body (10) has a thickness of between 4.0 mm and 12.0 mm.

4. Device according to claim 3, the thickness of said body (10) being decreasing from the end (201) embodying the square angle towards the end bearing the arc segment (401).

5. Device according to any one of the preceding claims 1 to 4, wherein the side walls (211, 311, 411, 511) are tapered from the base wall (111), towards the top wall (101).

6. Device according to claim 5, wherein the side walls (211, 311, 411, 511) of said body (10) are inclined, with respect to the axis perpendicular to the base wall, of an angle (α) between 30 ° and 45 °.

7. Series of therapeutic devices according to any one of claims 1 to 6, made for the right foot and the left foot of the patient, in which the oblique side (301) of trapezoidal rectangle corresponds substantially to the distance between the middle of plantar arch of the patient's foot and the articulation of the first metatarsal with the big toe, the greater base (201) corresponds to approximately 80% of this distance, the side (501) that concurs to form the square angle corresponds to about 95% of that distance, and the arc segment (401) has a radius of curvature (R) corresponding to approximately 30% of said distance, this distance being parameterized on the basis of the usual sizes of footwear on the market.
